Europäisches Patentamt

European Patent Office

Office européen des brevets

(19)

(11) Publication number: 0 298 620
A1

## (12) EUROPEAN PATENT APPLICATION

(21) Application number: 88305505.5

(22) Date of filing: 16.06.88

(51) Int. Cl.4 A61B 5/02

(30) Priority: 06.07.87 US 58975

(43) Date of publication of application:
11.01.89 Bulletin 89/02

(84) Designated Contracting States:
DE ES FR GB

(71) Applicant: The BOC Group, Inc.
85 Chestnut Ridge Road
Montvale, New Jersey 07645(US)

(72) Inventor: Susi, Roger E.
7006 E 89th Place
Tulsa Oklahoma 74133(US)
Inventor: Spraker, Terry E.
6391 S Geneva Circle
Englewood Colorado 80111(US)
Inventor: Morris, Timothy J.
13144 Saturn Drive
Littleton Colorado 80124(US)

(74) Representative: Gough, Peter et al
c/o THE BOC GROUP PLC Patent Department
Chertsey Road
Windlesham Surrey GU20 6HJ(GB)

(54) Blood pressure monitoring methods and apparatus.

(57) A blood pressure monitoring instrument monitors both systolic and diastolic blood pressures in a normal mode of operation, but monitors only systolic blood pressure in a stat mode to provide a rapid indication of blood pressure, and to provide a series of blood pressure readings in rapid succession. A plethysmographic signal can be used to detect return to pulsatile flow as the cuff or occluding pressure decreases during stat mode operation.

EP 0 298 620 A1

FIG. 1

# BLOOD PRESSURE MONITORING METHODS AND APPARATUS

The present invention relates to medical instrumentation and particularly relates to blood pressure monitoring methods and apparatus.

The blood pressure and pulse of a living subject may be monitored during medical procedures such as surgical operations and the like. Conventional monitoring instruments includes means for automatically taking oscillometric blood pressure readings. Thus, the instrument typically includes a cuff, a gas pressure pump for inflating the cuff and a pressure sensor connected to the cuff. In use, the cuff is applied to a limb of the subject and automatically inflated and deflated so that the pressure within the cuff varies from above the systolic blood pressure to below the diastolic blood pressure. As the inflation pressure decreases to the systolic blood pressure, the fluid pressure signal provided by the sensor begins to oscillate. The apparatus includes means for detecting onset of these oscillations in the fluid pressure signal and capturing the fluid pressure prevailing at the onset of such oscillations as the systolic blood pressure of the subject. When the fluid pressure within the cuff declines further, to the subject's diastolic blood pressure, the magnitude of these oscillations decreases markedly. In response to this further change, the apparatus captures the prevailing pressure within the cuff as the patient's diastolic blood pressure. Typically, these measurement cycles are repeated at predetermined intervals, as, for example, every few minutes. The apparatus also may be adapted to repeat the oscillometric blood pressure measurement cycle on demand, in response to a manual actuation. These systolic and diastolic blood pressures together provide the physician with the complete data needed for monitoring the patient's condition over the long term, and the oscillometric measurement method ordinarily provides accurate values.

However, the cuff must be deflated progressively, at a controlled slow rate during this oscillometric measurement cycle, and the complete measurement cycle required to determine both the systolic and diastolic pressures requires a considerable interval. The response time of the apparatus after manual actuation is at least equal to this interval. Also, the time between successive blood pressure measurements is at least equal to this interval. In an emergency, the physician may need an immediate blood pressure reading and/or a series of blood pressure readings taken in rapid succession.

There have accordingly been unmet needs heretofore for blood pressure monitoring apparatus and methods which can provide an immediate indication of blood pressure when needed, and which can provide blood pressure readings in rapid succession. There have been particular needs for apparatus and methods which can provide complete blood pressure indications suitable for long-term patient monitoring, such as indications of both the systolic and diastolic blood pressures, but which can also provide an indication of blood pressure immediately when needed.

The present invention addresses those needs.

According to one aspect of the present invention, apparatus for monitoring blood pressure in a subject is characterised by

(a) pressure application means for applying an occluding pressure to a predetermined pressure application region of the body of said subject;

(b) transducer means for generating one or more signals representative of blood flow through said pressure application region;

(c) signal processor means for monitoring at least one of said one or more signals;

(d) control means for automatically actuating said pressure application means, transducer means and signal processor means so as to determine only the systolic blood pressure of said subject.

Preferred embodiments of apparatus according to this aspect of the invention can provide the complete, accurate blood pressure information needed for long term monitoring, and can also provide rapid blood pressure indications as needed to monitor rapid changes in the subject's condition.

Apparatus according to this aspect of the present invention preferably includes pressure application means, such as an inflatable cuff, for applying an occluding pressure to a pressure application region of the subject's body, typically the upper arm. Transducer means are provided for generating one or more signals representative of blood flow through that pressure application region. Signal processor means are provided for monitoring the signal or signals generated by the transducer means. The apparatus preferably also includes control means having a normal mode and a stat mode, and manually actuable selection means for selecting the stat mode when required. In the normal mode, the control means is operative to actuate the pressure application means, transducer means and signal processor means so as to determine both the systolic and diastolic pressures. In the stat mode, the control means automatically actuates the pressure application means, transducer means and signal processor means so as to determine only one of these blood pressures. Most preferably the systolic blood pressure is determined repeatedly in the stat mode.

On each repetition of the systolic blood pressure measurement in the stat mode, the occluding pressure may be adjusted to a starting pressure differing from the systolic pressure and the occluding pressure may then be varied until the signals generated by the transducer means indicate a change in the blood flow, whereupon the prevailing occluding pressure can be captured and displayed as the systolic pressure. Most preferably, the starting pressure used on each systolic pressure determination in the stat mode is just slightly above the systolic pressure, so that blood flow is substantially occluded when the occluding pressure is at the starting point. The signal processor means may be arranged to detect a change in the signal from the transducer means which indicates resumption of blood flow and capture the occluding pressure prevailing at the time of such resumption as the systolic pressure.

In the stat mode, the pressure need not vary at a progressive, limited rate over the entire range encompassing the systolic and diastolic pressure. Rather, the pressure need only vary progressively over the relatively narrow range from the starting pressure to the actual systolic pressure. Therefore, the instrument can provide systolic pressure readings in rapid succession.

The pressure applying means preferably includes a device such as a cuff having a distensible pressure applying member and means for providing fluid under pressure within the distensible member. The transducer means may include a fluid pressure transducer connected to the distensible member. In the normal mode, the signal processing means may detect oscillations in the fluid pressure signal from this transducer to perform an oscillometric blood pressure determination. Preferably, the transducer means also includes an additional transducer for detecting changes in blood flow independently of the fluid pressure signal. Thus, the transducer means may include plethysmograph means for providing a plethysmograph signal representing blood volume within an anatomical feature of the subject, such as a fingertip, distal to the region engaged with the pressure applying means. Oscillations in the plethysmograph signal will indicate occurrence of pulsatile blood flow through the portion of the subject engaged by the pressure applying means. The signal processing means may be arranged to detect oscillations in the plethysmograph signal and capture the occluding pressure of fluid pressure in the cuff prevailing upon commencement of these oscillations as the systolic blood pressure. Because the stat mode systolic pressure determination does not depend upon sensing oscillations in the occluding pressure or cuff fluid pressure, the occluding pressure can be decreased rapidly without causing spurious signals. Thus, the system can provide a blood pressure reading promptly after it enters the stat mode.

The plethysmograph means may include a simple light source and photodetector assembly together with appropriate devices for amplifying the signal from the photodetector. The plethysmograph means may also serve as part of a pulse measuring system. Most preferably, the apparatus automatically adjusts the amplifier gain to provide an amplified signal of the desired amplitude and establishes an average value for the pulse period and tests the amplified plethysmograph signal while monitoring the pulse during dwell periods between pressure measurements. The system may include safety features to prevent stat rode operation if a good plethysmograph signal cannot be obtained. Also, the average pulse period can be used as a standard to reject invalid plethysmograph signals in the stat mode. The control means and signal processor means may be provided as elements of a microprocessor or microcomputer, and the same microprocessor or microcomputer can be employed to perform other machine operations as well. The same pressure application means as utilized for the normal mode determinations can be used for stat mode operation as well. Thus, the stat mode capability can be provided in the apparatus at only a minimal cost. Moreover, this added function is achieved without adding yet another instrument to the operating room environment.

According to a further aspect of the present invention, a method of monitoring blood pressure of a subject is characterised by the steps of:

(a) automatically monitoring both the systolic and diastolic blood pressures of said subject in a normal mode of operation; and

(b) interrupting said normal mode of operation in response to a manually applied actuation; and

(c) automatically determining only the systolic blood pressure of said subject in a stat mode of operation when said normal mode of operation is interrupted in response to said manually applied actuation.

In the preferred methods, both the systolic and diastolic pressures are monitored in a normal mode, whereas only the systolic pressure is monitored in the stat mode, using the same pressure application means or cuff as employed for the normal mode monitoring step. Monitoring methods according to this aspect of the present invention provide benefits similar to those discussed above in connection with the apparatus.

An embodiment of the invention will now be described, by way of example, reference being made to the Figures of the accompanying diagrammatic drawings in which:

Figure 1 is a schematic perspective view of apparatus according to one embodiment of the present invention.

Figure 2 is a schematic view, partially in block diagram form, of the apparatus shown in Figure 1.

Figures 3 and 4 are each simplified logic diagrams illustrating steps in operation of the apparatus shown in Figs. 1 and 2.

Apparatus according to one embodiment of the present invention includes a housing 10 with a systolic blood pressure display 12, mean blood pressure display 14, diastolic blood pressure display 16 and pulse rate display 18 mounted to a front panel. These displays are all digital displays. A "stat" or immediate blood pressure reading button 22 is also mounted on the front panel of housing 10, along with other operating controls (not shown).

The apparatus also includes a cuff 24 arranged for mounting on the arm of a human patient or subject P. Cuff 24 incorporates a distensible bladder 26 connected via a supply tube 28 to a pump 30 and valve 32, and to a pressure transducer 34. Pump 30 and valve 32 are connected via conventional interfacing circuits 36 and 38 to the central microprocessor 40 of the apparatus. Pressure transducer 34 is also connected via conventional interfacing circuitry 46 to the master microprocessor 40. Microprocessor 40 is arranged to perform both control and signal processing functions as described hereinbelow, and accordingly is shown as including a control portion 42 and a signal processing portion 44. As will be appreciated, this block functional depiction is merely for purposes of illustration. As is conventional in microprocessor structures, many of the same components perform both control and signal processing operations at different times.

Microprocessor 40 is linked to a momentary switch 48 which is mechanically connected to stat pressure reading button 22, and the microprocessor 40 is also connected to each of the displays 12, 14, 16 and 18.

The apparatus also includes a fingertip probe 50 adapted to engage the tip of the subject's finger F on the same arm where the cuff 24 is mounted. Fingertip probe 50 includes an infrared light emitting diode 54, a red light emitting diode 52 and a photodetector 55. The light emitting diodes 52 and 54, and the photodetector 55 are mounted within fingertip probe 50 so that the light emitted by diodes 52 and 54 passes along a predetermined light path and impinges upon detector 55. Probe 50 also includes conventional devices 53 for holding the probe on the finger F without obstructing blood flow to the fingertip.

Diodes 52 and 54 are linked via leads 56 and 58 to conventional power supply circuits 62 and 64 controlled by microprocessor 40. Leads 56 and 58 are incorporated in a multi-lead cable 60 connecting fingertip probe 50 and housing 10. A further lead 66 of cable 60 connects the photodetector 55 of fingertip probe 50 to photodetector interface 68. The photodetector interface includes a low pass filter 70 connected directly to the output of the photodetector and a fixed-gain operational amplifier 72 connected to the output of filter 70. The output of operational amplifier 72 is in turn connected through a band pass filter network 74 to the input of a further operational amplifier 76. Amplifier 76 converts the single ended output of amplifier 72 and filter 74 into a double-ended signal, and delivers this double-ended signal to the input of an electrooptical isolation device 78. The outputs of isolation device 78 are connected to the inputs of a further fixed gain operational amplifier 80. The output of amplifier 80 is connected via a further high pass filter 82 to the input of an adjustable gain amplifier 84. The gain of amplifier 84 is controlled through a gain adjustment terminal 86 which is connected via a digital to analog converter 88, to an output of microprocessor 40. The signal output of amplifier 84 is connected, via an analog-to-digital converter 90, to an input of the microprocessor 40.

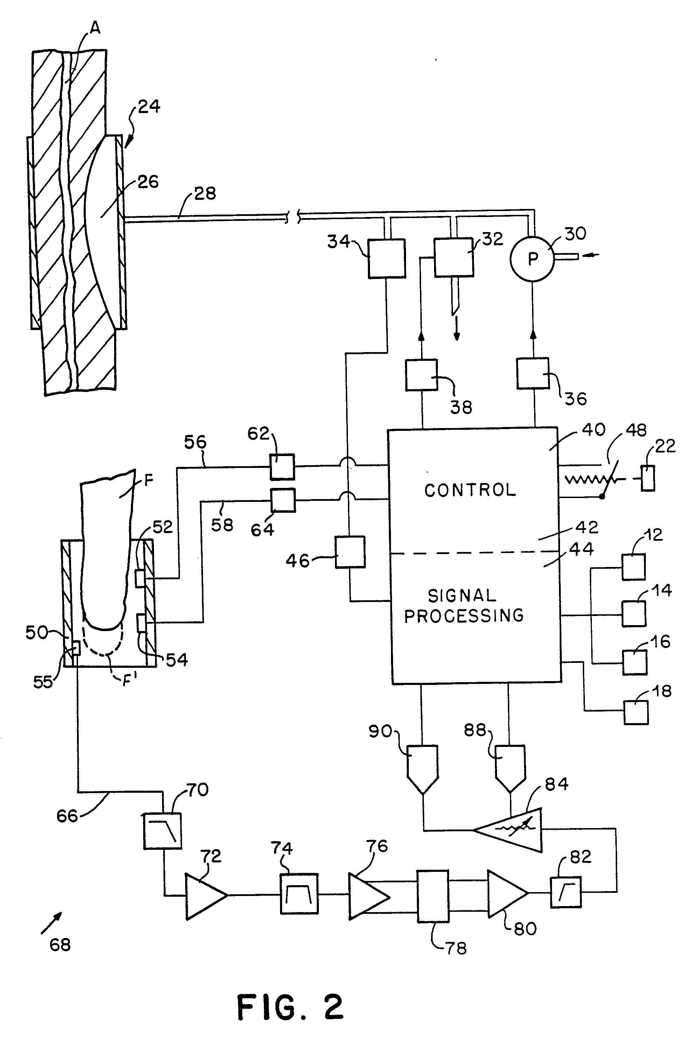

In a monitoring method according to one embodiment of the invention, the cuff 24 is mounted to the upper arm of the subject or patient P, whereas the fingertip probe 50 is mounted to the patient's fingertip F. As shown in Figure 2, the patient's fingertip is disposed between the infrared light emitting diode 54 and photodetector 55, so that the patient's fingertip F occludes the light path between diode 54 and the photodetector. While the distensible bladder or member 26 of cuff 24 is in a deflated condition, the patient's normal pulse causes periodic expansion and contraction of the small arteries within fingertip F. Thus, the volume of arterial blood within fingertip F changes periodically. Absorption of the infrared light impinging on photodetector 55, varies with the volume of arterial blood in fingertip F. Therefore, while the light emitting diodes 52 and 54 are continuously illuminated, the output of photodetector 55 will vary periodically in accordance with the periodic changes in the arterial blood volume of fingertip F induced by the pulse. The red light from diode 52 indicates that the fingertip probe is operating.

This periodically varying component, having the same fundamental frequency as the pulse rate of the subject (typically on the order of about 1-2Hz) passes through low pass filter 70 amplifier 72 and band pass filter 74, which has a pass band from about 0.2Hz to about 5Hz. The filtered signal is converted to a voltage difference by amplifier 76, and passed to isolator 78. The isolator is arranged to convert the voltage difference to light and then back to a further output voltage difference signal. Thus, the isolator 78 serves to pass the signal from the output of amplifier 76 to the input of amplifier 80, without any direct electrical interconnection between these two amplifiers. High pass filter 82 removes DC or low frequency components which might be added to the signal by isolator 78. The filtered and amplified signal passing from amplifier 80 via high pass filter 82 is further amplified in variable gain amplifier 84, and passes to the signal processing components of master microprocessor 40 via analog to digital converter 90.

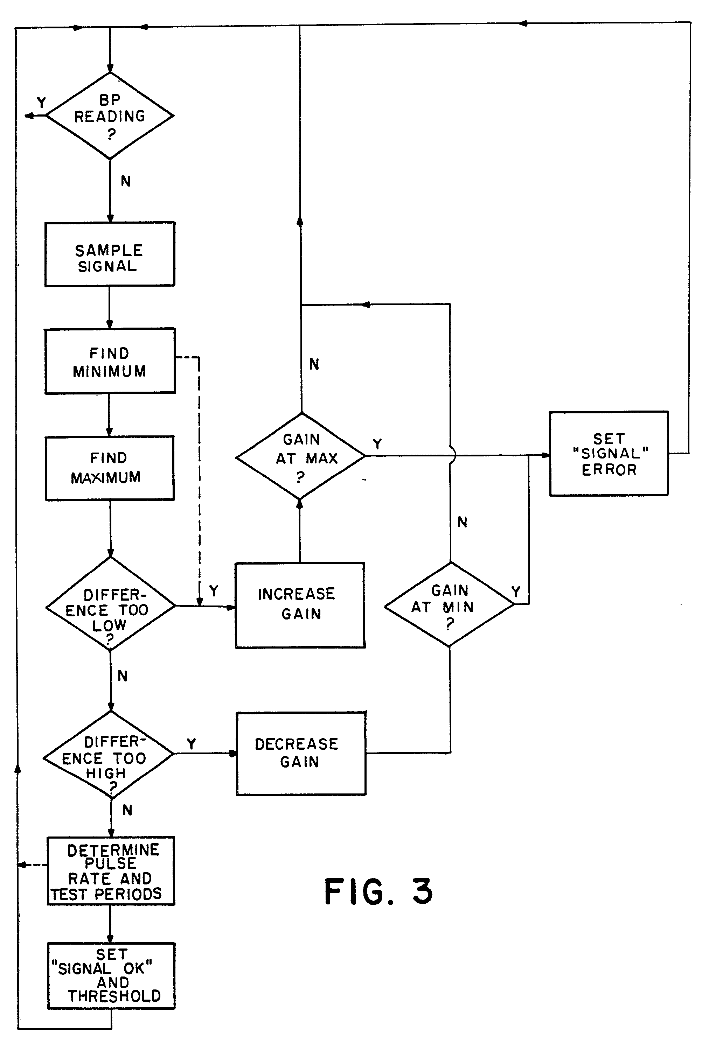

The microprocessor 40 controls the operation of variable gain amplifier 84 so as to assure that the periodic or pulse wave signals passing into the microprocessor via converter 90 are within a predetermined, relatively narrow range of amplitudes despite physiological variations and variations in the placement and/or response of the fingertip probe 50. Also, the microprocessor extracts pulse rate information from this periodic signal. The logic of the microprocessor operations for performing this function is schematically illustrated in Figure 3. At decision block 100, the microprocessor will exit from the pulse determination and gain control routine if a blood pressure reading is required. If not, the microprocessor proceeds to sample the signal entering via analog to digital converter 90, representing the output of photodetector 55. At this stage, the gain of amplifier 84 is atadefault value. As no blood pressure reading is being taken, distensible member 26 of cuff 24 is deflected, and the artery A will be substantially unoccluded. There will be a good pulse at fingertip F, and hence the amplified photodetector output signal passing to microprocessor 40 from amplifier 84 via converter 90 will have periodically varying values corresponding substantially to the pulse of the subject, with a maximum and minimum on each cardiac cycle of the subject.

The microprocessor finds an oscillation with a relatively rapid rate of change or high first derivative, and finds the minimum associated with this oscillation in the amplified photodetector signal, by checking the value of the signal at successive sampling intervals. When the value of the amplified photodetector output signal increases by more than a predetermined threshold amount between successive sampling intervals, the oscillation is recognized as valid and the value at the earlier one of these two successive sampling intervals is taken as the minimal value associated with the oscillation. Thus, the difference between the value of the amplified photodetector signal is taken as approximating the first derivative of the amplified photodetector signal. Oscillations representing true pulse waveforms will normally have a high first derivative.

The system will not pass to the steps of identifying minimum or maximum values associated with an oscillation in the signal until it finds an oscillation with a first derivative meeting the required threshold. Thus, the system will reject slow oscillations which result from factors other than pulses.

If no such possibly valid oscillation occurs within a predetermined time period, the system passes directly to the increase gain subroutine discussed below as indicated in broken lines. If the system finds a valid oscillation with the requisite first derivative and finds the minimum associated with that oscillation, the system proceeds to find the maximum value associated with that oscillation. Thus, the microprocessor compares successive sampled values of the signal following the minimum. A value followed by a lower value is taken as the maximum value. The microprocessor compares the minimum and maximum values determined in this fashion and computes the difference between them.

The difference between the minimum and maximum values of the amplified photodetector output signal represents the amplitude of the oscillation. If this amplitude is below a predetermined minimum, the microprocessor transfers to the increase gain subroutine. Thus, the increase gain subroutine is called either if the microprocessor does not detect any oscillation with the threshold first derivative, and hence does not find a minimum value, or if the microprocessor finds both minimum and maximum values but the amplitude of the oscillation is below the desired aptitude.

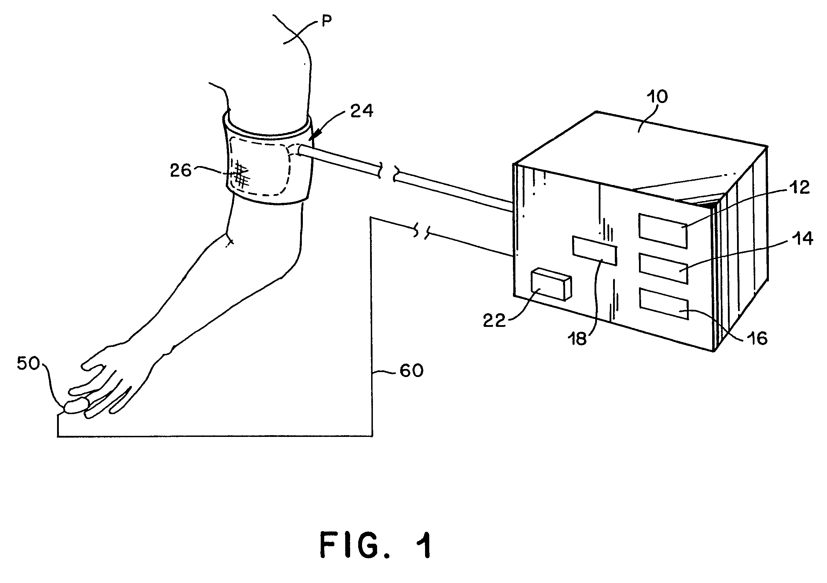

In the increase gain subroutine, the microprocessor sends a signal via digital to analog converter 88 to the control input 86 of variable gain amplifier 84 (Fig. 2) to raise the gain of the amplifier. The microprocessor then tests to see whether the gain of this amplifier is at a preestablished maximum value. If so, the system sets a signal error flag indicating that even with maximum gain in the amplification system, the photodetector and associated electronic components will not produce the desired periodically varying signal. Such a condition may occur for example, if the fingertip probe 50 (Figs. 1 and 2) is disconnected, or if the fingertip probe is mounted improperly, so that the fingertip is misaligned with the photodetector. However, if the gain is not at the preestablished maximum, the system returns to its starting condition without setting the error flag. In either case, the system repeats the foregoing sequence of operations.

If the difference between the minimum and maximum values exceeds the preestablished minimum, the microprocessor does not enter the increase gain subroutine but instead proceeds to test the difference or amplitude against a preestablished maximum. If the difference exceeds the preestablished maximum, the microprocessor passes to a decrease gain subroutine, and signals the amplifier 84, via digital to analog converter 88 and control input 86 to decrease the gain. The system then tests the gain to see if the gain has been reduced to a preestablished minimum. If so, the system sets the signal error flag, indicating that the amplified fingertip probe signal has an amplitude greater than the maximum allowable amplitude even with the gain in the system set to the minimum. Such a condition may occur if the photodetector probe signal is subject to spurious variations due to electromagnetic interference, movement of the fingertip probe with respect to the patient's fingertip or the like. Again, whether or not the system sets a signal error flag, it will return to the starting point of this sequence of operations and repeat the sequence again.

If the amplitude of the oscillation in the amplified photodetector signal is in the desired range, between the predetermined minimum and maximum values, the microprocessor will pass to the pulse rate determination step, and determine the pulse rate by the time elapsed between one maximum in the amplified photodetector output signal and the next succeeding maximum. This period corresponds to the period of the patient's pulse. The system computes an average of the period for the last few oscillations. By comparing each newly found period to this average, the system determines whether the newly found period corresponds to the average within a predetermined tolerance, typically plus or minus 18% of the average. If it does not, the system rejects the last oscillation as a spurious, non-periodic signal, sets the signal error flag returns and again to the starting point without returning a pulse rate value, as indicated in broken lines in Figure 3. If the period of the last oscillation does correspond with the average, then the system treats the period between the last oscillation and the preceding oscillation as a valid pulse period and includes this last period in the average.

When valid periods are detected in this fashion, the system sets a signal OK flag and clears any signal error flag to indicate that the fingertip probe 50 and associate circuitry are functioning properly. In this "signal OK" condition, the microprocessor computes values for the average amplitude and threshold (first derivative) of the oscillations in the amplified photodetector signal representing valid pulses. In this condition, with the "signal OK" flag set, the microprocessor will compute the pulse rate and display the pulse rate on display 18. By contrast, in the "signal error" condition with the "signal error" flag set and the "signal OK" flag not set, the microprocessor shows a warning symbol (such as a series of dashes) on the pulse rate display 18. The system returns to the start of this pulse detection routine and continuously repeats the sequence provided that no blood pressure reading is required at the time. The microprocessor thus continually updates both the pulse data and the condition of the photodetector signal (OK or error).

In the normal operation mode, the apparatus takes oscillometric blood pressure readings at predetermined intervals as timed by microprocessor 40. The apparatus may be provided with controls (not shown) for adjusting the length of these intervals. When such an interval has elapsed, and a new reading is required, microprocessor 40 commands valve 32 to close and commands pump 30 to pump air into the distensible member 26 of the cuff. During this cycle, the system will pump air into distensible member 26 until the pressure reaches a starting value which should be sufficient to substantially occlude artery A and block blood flow through this artery. If a systolic pressure reading has been taken within the last few minutes, the system will use this last systolic pressure, plus a predetermined margin, as the starting value. If no systolic pressure has been determined, the system will employ a default value greater than typical normal systolic pressures, and most preferably about 170 mm Hg, as the starting value. Once the system reaches the starting value, the pump is shut off and the system monitors the signal from pressure transducer 34 to determine the magnitude of oscillations in this signal.

The system periodically opens valve 32 so as to bleed air from distensible member 26 and decrease the occluding pressure applied to the subject's arm stepwise. After each such stepwise decrease, the system determines the magnitude of oscillations in the signal from pressure transducer 34. When the pressure in distensible member 26, and hence the occluding pressure applied to the subject's arm, is equal to the subject's systolic blood pressure, pulsatile flow through artery A will commence once again. This pulsatile flow will cause pulsations in the air within distensible member 26 and hence will cause a substantial increase in the amplitude of oscillations in the signal from pressure transducer 34. The microprocessor captures the pressure prevailing at this increase in magnitude of the oscillations as the patient's systolic blood pressure, and shows the systolic blood pressure on systolic blood pressure display 12.

If the amplitude of the oscillations in the signal from pressure transducer 34 does not increase appreciably after several stepwise decreases in the pressure within member 26, the microprocessor takes this as a signal that the starting pressure was insufficient to occlude artery A. The microprocessor actuates pump 30 and valve 32 to increase the pressure within distensible member 26 once again to a new starting value, about 60 mm Hg higher than the prior starting value. The system repeats the cycle of stepwise deflation and testing for an increase in amplitude of oscillations in the cuff pressure signal, until the systolic pressure is found. If no systolic pressure is found after several deflation steps, the starting pressure is increased once again. If a fault in the system prevents detection of the systolic pressure the pump 30 could continually inflate the distensible member 26 to progressively higher starting pressures. An overpressure safety switch (not shown) in communication with member 26 will shut the system off and release the pressure within member 26 if the pressure exceeds a predetermined maximum value.

After the systolic pressure has been found, the microprocessor continues actuation of valve 32 to continue bleeding air from the distensible member. When the occluding pressure reaches the patient's diastolic blood pressure, the magnitude of the oscillations in the signal from transducer 34 decreases markedly. The occluding pressure prevailing in the distensible member of the cuff at this time is captured by the microprocessor as the subject's diastolic blood pressure and shown on diastolic blood pressure display 16. The microprocessor also computes the subject's mean blood pressure and shows the same on mean pressure display 14. The microprocessor also compares the systolic, diastolic and mean pressure values with appropriate limits set by the operator through limit adjustment controls (not shown) and actuates an alarm if the actual pressures deviate from the set limits.

In normal operation, this cycle of operations is repeated periodically, so as to repeat the oscillometric blood pressure determination at the required intervals. The figures shown in the blood pressure displays are updated on each repetition. During the intervals between oscillometric blood pressure determinations in normal mode operation, valve 32 is open and the air is released from distensible member 26, so as to release the occluding pressure on the subject's upper arm. During these intervals, the pulse rate determination cycle discussed above is repeated. The system may also be provided with controls (not shown) for selecting a "hold" mode, in which the oscillometric pressure determinations are not repeated. In the hold mode, the last measured values would be continually displayed. Also, the system may be provided with controls to allow the operator to override the automatic cycle and control the occluding pressure directly. In this manual mode, the system operates as a manual sphygomanometer. Ordinarily, the physician would use this capability to perform a manual blood pressure determination, and would use an instrument such as a stethoscope for monitoring the flow through artery A.

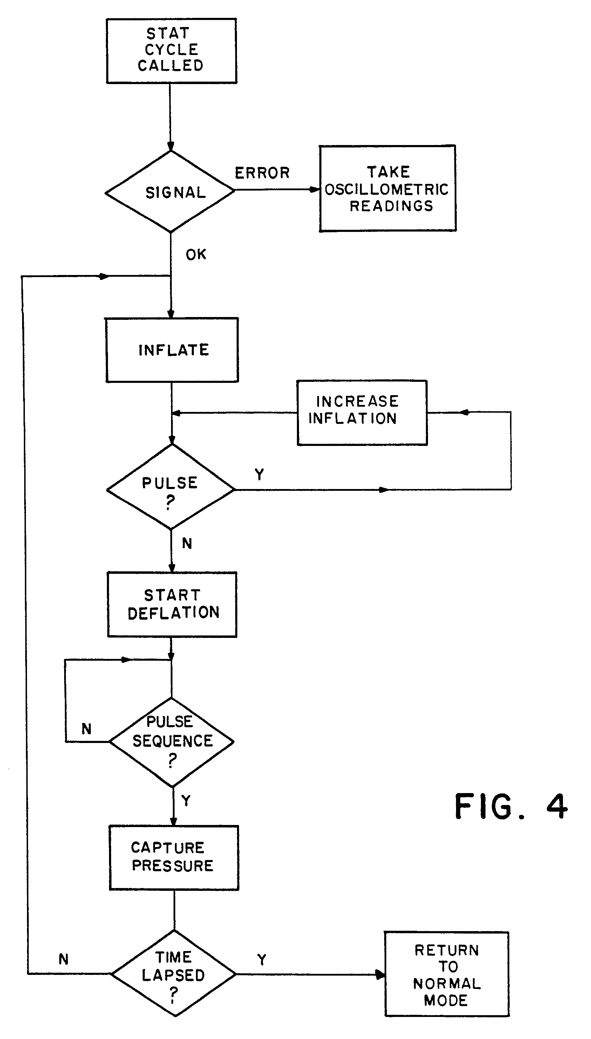

When immediate blood pressure information is required, the operator need only touch the "stat" button 22 (Figs. 1 and 2), thereby actuating momentary switch 48. In response to actuation of the momentary switch, microprocessor 40 enters the stat routine depicted in Fig. 4. This routine uses the plethysmographic signal from fingertip probe 50 and the associated circuitry to detect pulsatile flow. Because the fingertip F monitored by probe 50 is distal to the upper arm engaged by cuff 24, the fingertip is supplied with blood through that region of artery A occluded by the occluding pressure in the distensible member 26 of the cuff. Therefore, when artery A is fully occluded by the pressure applied through the cuff, there will be no pulse at fingertip F, and the periodic component of the signal from the photodetector will disappear. When the occlusion is released, and the occluding pressure falls to the patient's systolic pressure, the pulse will return at fingertip F and the periodic variation in the plethysmographic signal will resume.

As the first step in the stat cycle, the system tests the plethysmographic signal by checking the "signal OK" and "signal error" flags set during the pulse monitoring routine. If the signal OK flag is not set and the signal error flag is set, these indicate that the plethysmographic signal is unreliable and cannot be used to detect the pulse. Rather than attempting to execute the remain der of the stat cycle, the system will automatically take an oscillametric blood pressure reading which, as discussed above, does not utilize the plethysmographic signal to determine pressure. This provides a significant "fail safe" protection for the patient.

If the signal OK flag is set, and the signal error flag is not set, then the plethysmographic signal is reliable, and the microprocessor proceeds to the remainder of the stat cycle. During this cycle, the gain applied in amplifying the plethysmographic signal from photodetector 55 is fixed at the value established during the pulse-detection operations discussed above.

The microprocessor begins timing a predetermined stat cycle interval, actuates valve 32 (Fig. 2) to close and actuates pump 30 to inflate the distensible member 26 in cuff 24 to a starting occluding pressure. The starting pressure will be approximately 25-30 mm Hg above the last measured systolic pressure, or will be the default value if no systolic pressure has been determined within the last few minutes.

After the occluding pressure reaches the starting pressure, the system tests the plethysmographic signal (the amplified signal from photodetector 55) to determine whether or not a pulse is present at fingertip F. The system checks for an oscillation with the high first derivative characteristic of a true pulse waveform. Thus, the microprocessor samples the amplified photodetector signal periodically and tests for successive samples differing from one another by at least the threshold value established during the pulse detection routine. If no such pair of successive sampled values is found within a predetermined time, then there is no pulse. If there is such a pair of successive sampled values, the micro processor treats that set of successive values as indicating the minimum of the oscillation and proceeds to check for the maximum value of the amplified photodetector signal. The microprocessor then subtracts the minimum value from the maximum value to determine the amplitude of the pulse. Only if the pulse exceeds the minimum amplitude likewise established during the pulse monitoring routine does the system regard a variation in the amplified photodetector output signal as showing a true pulse. As will be appreciated, the gain setting features of the pulse monitoring routine are not employed during this routine. The gain of amplifier 84 is fixed during the entire stat cycle.

If the system finds a valid pulse in this fashion, it will actuate pump 30 to increase the occluding pressure within distensible member 26 by a predetermined amount and then repeat the signal monitoring sequence. Thus, if the initial or starting pressure 160 is less than the systolic pressure prevailing at the time, the system will detect pulses at fingertip F and will increase the occluding pressure until the pulses disappear and a predetermined time elapses without the occurrence of a further valid pulse.

After a predetermined time elapses without occurrence of a valid pulse, as indicated by the photodetector or plethysmographic signal, microprocessor 40 starts a deflation routine. In the deflation routine, the microprocessor opens and closes valve 32 at a rapid rate, and checks the occluding pressure level or level of fluid pressure in distensible member 26 by monitoring the signal from pressure transducer 34. The microprocessor monitors the rate of deflation or rate of pressure decrease and adjusts the duty cycle or percentage of time valve 32 is open to keep the average deflation rate in a predetermined range, preferably at least about 3mm Hg/sec and most preferably between about 3 and about 6mm Hg/sec, so that the cuff deflates rapidly.

While the cuff deflates, the microprocessor continues to monitor the signal from the photodetector. In this pulse detection routine as well, the system uses the same criteria to detect variations in the plethysmographic or photodetector signal which may indicate a valid pulse, viz, a minimum rate of change or difference between successive samples, and a pulse amplitude or difference between minimum and maximum values corresponding, within a predetermined tolerance, to the average pulse amplitude set in the pulse monitoring routine. When a single seemingly valid pulse signal meeting these criteria is detected, the microprocessor temporarily captures the then prevailing value of the occluding pressure, as monitored by transducer 34, as a possible value of the subject's systolic pressure.

However, this value is not treated as a valid systolic pressure value until another seemingly valid pulse has also been detected. The period between these two successive seemingly valid pulses is compared with the average pulse period established during the pulse monitoring routine. If the period between pulses matches the average pulse period within a predetermined tolerance, typically plus or minus 18 percent, then the two successive seemingly valid pulse signals are treated as representing true pulses. The possible systolic pressure value captured on the occurrence of the first apparent pulse signal is finally captured as a value of the patient's systolic pressure and shown on the systolic pressure display 12. If the sequence of two apparently valid pulse signals, with the appropriate period between pulse signals, does not occur, the microprocessor disregards the temporarily captured value and continues to monitor the plethysmographic or photodetector signal while the cuff continues to deflate.

As soon as a valid systolic pressure value has been captured, the system checks the timer started at the beginning of the stat routine. If the predetermined stat cycle interval has not elapsed, the microprocessor immediately terminates the deflation cycle and reinflates the distensible member of the cuff to a new starting pressure higher than the captured systolic pressure by a predetermined amount typically about 25-30 mm Hg. The systolic pressure determination is repeated once again. Again, the microprocessor will test the plethysmographic signal for possibly valid pulse signals and, if none are observed, will start the deflation cycle. During this repeated deflation cycle, the microprocessor again tests the plethysmographic signal to detect variations which apparently indicate a valid pulse at fingertip F. Once a sequence of two apparent pulse signals is detected again, a new value of the systolic pressure will be captured again as described above and the displayed systolic pressure will be updated to reflect the new value. This cycle of operations will be repeated again and again until the stat cycle interval elapses, whereupon the system will return to normal mode operation. Upon the initial return to normal mode, the cuff is deflated to restore circulation through the arm bearing cuff 24, and then the cycle of oscillametric blood pressure readings at normal intervals, and pulse rate monitoring is resumed.

During the stat cycle interval, the system will provide repeated systolic pressure readings in rapid succession, typically about one reading every 15 seconds. Because the occluding pressure need only be cycled in a relatively narrow range from just above the prevailing systolic pressure to just below the systolic pressure, the relatively long interval required for gradual reduction of the pressure over the entire blood pressure range, encompassing both the systolic and diastolic pressures, is eliminated. Also, because the pulse is detected by the fingertip probe, independently of any oscillations in the pressure transducer signal, the deflation or reduction in occluding pressure can proceed at a rapid rate. Thus, there is no need to stop the deflation cycle and check for oscillations in the pressure transducer signal. When this stat cycle interval has elapsed, the microprocessor returns to normal mode operation. Upon the initial return to normal mode, the cuff is deflated to restore circulation through the arm bearing cuff 24, and then the cycle of oscillometric blood pressure readings at normal intervals, and pulse rate monitoring is resumed.

Numerous variations and combinations of the features described above can be utilized without departing from the present invention. Thus, the plethysmographic fingertip probe used to detect pulse rate and to detect pulse during the stat cycle could be replaced by a different form of transducer such as an electronic stethoscope or the like. In a distinctly less preferred variant, oscillometric monitoring could be employed in the stat cycle as well as in the normal cycle. In this variant, the occluding pressure within member 26 of the cuff could be decreased stepwise from the starting point in measuring the systolic pressure. An increase in amplitude of oscillations in the signal from transducer 34 would signal resumption of flow through artery A. This variant is less preferred inasmuch as it would require that the system stop the deflation or pressure decrease cycle on each step for a predetermined time and then wait to detect the magnitude of oscillations in the pressure transducer signal.

In a further variant, the normal mode pressure determinations can be omitted. Thus, the apparatus and methods described above can provide systolic pressure determination only, without any normal mode systolic and diastolic pressure monitoring cycle. In this variant, the systolic pressure may be repeatedly determined during pressure monitoring intervals by the same technique as described above for stat mode operation. The cuff may remain deflated between pressure monitoring intervals, and the gain of the plethysmograph signal amplifier can be adjusted as described above while the cuff is deflated. Each pressure monitoring interval may be initiated manually or in response to a timer of other controlling device.

As these and other variations and combinations of the features described can be utilized without departing from the present invention as defined in the claims, the foregoing description of the preferred embodiments should be taken by way of illustration rather than by way of limitation of the present invention.

## Claims

1. Apparatus for monitoring blood pressure in a subject characterised by:

(a) pressure application means 24, 26 for applying an occluding pressure to a predetermined pressure application region of the body of said subject;

(b) transducer means 34, 50 for generating one or more signals representative of blood flow through said pressure application region;

(c) signal processor means 44 for monitoring at least one of said one or more signals; and

(d) control means 42 having a stat mode for automatically actuating said pressure application means 24, 26, transducer means 34, 50 and signal processor means 44 so as to determine only the systolic blood pressure of said subject.

2. Apparatus as claimed in claim 1, characterised in that:

said control means 42 has a normal mode and a stat mode, said control means 42 being operative in said normal mode to actuate said pressure application means 24, 26, transducer means 44 and signal processor means so as to determine both the systolic and diastolic blood pressures of said subject, said control means 42 being operative in said stat mode to automatically actuate said pressure application means 24, 26, transducer means 34 and signal processor means 44 so as to determine only one of said blood pressures of said subject; and

selector means 22 for selecting said stat mode upon manual actuation.

3. Apparatus as claimed in Claim 2, characterised in that said control means 40 is operative in said stat mode to actuate said pressure application means 24, 26, transducer means 34 and signal processor means 44 so as to determine only the systolic blood pressure of said subject.

4. Apparatus as claimed in Claim 1, 2 or 3 characterised in that said control means 42 is operative in said stat mode to actuate said pressure application means 24, 26 so that said occluding pressure decreases progressively from above the systolic blood pressure of said subject, whereby said blood flow through said portion of the subject's body will be interrupted while said occluding pressure is above said systolic pressure, and wherein said signal processor means 44 includes means for capturing the occluding pressure prevailing when said one or more signal indicates that said blood flow has resumed.

5. Apparatus as claimed in Claim 4, characterised in that said control means 40 includes means for actuating said pressure application means to increase said occluding pressure for a further repetition of said systolic pressure determination in response to capture of said occluding pressure as said systolic pressure.

6. Apparatus as claimed in any one of Claims 1 to 5, characterised in that said transducer means 34 includes pressure transducer means 34 for providing an occluding pressure signal representative of said occluding pressure applied b said pressure application means 24, 26 and independent transducer means 50 for providing a signal indicating blood flow through said pressure application region independently of said occluding pressure signal.

7. Apparatus as claimed in Claim 6, characterised in that said independent transducer means includes means 50 for monitoring a distal region of said subject's body distal to said pressure application region.

8. Apparatus as claimed in Claim 7, characterised in that said independent transducer means includes plethysmograph means 54, 55 for providing a plethysmograph signal representing the blood volume of said distal region of said subject whereby oscillations in said plethysmograph signal will indicate occurrence of pulsatile blood flow through said pressure application region of said subject, and said means for capturing includes means for detecting oscillations in said plethysmograph signal during said progressive decrease in said occluding pressure and means for capturing the occluding pressure prevailing upon commencement of said oscillations and providing the captured pressure as said systolic blood pressure.

9. Apparatus as claimed in Claim 8, characterised in that said plethysmograph means includes a light source 54, a photodetector 55, and means 53 for fastening said light source 54 and said photodetector 55 to an extremity of said subject.

10. Apparatus as claimed in Claim 9, characterised by amplifier means 72 for amplifying said plethysmograph signal to provide an amplified plethysmograph signal, and gain adjust means for detecting the amplitude of oscillations in said plethysmograph signal and automatically adjusting the gain of said amplifier means to provide an amplified plethysmograph signal having oscillations within. a predetermined range of amplitudes while said occluding pressure is not applied, so that each pulse of said subject will result in an oscillation of said amplified plethysmograph signal within said predetermined range.

11. Apparatus as claimed in Claim 10 characterised by period determination means for determining the periods between successive oscillations in said amplified plethysmograph signal within said predetermined range of amplitudes while said occluding pressure is not applied.

12. Apparatus as claimed in Claim 11 characterised by means for limiting the range of adjustment of said gain of said amplifier means, and means for providing an error signal if adjustment of said gain within said range does not provide said oscillations in said amplified plethysmograph signal within said predetermined range of amplitudes while said occluding pressure is not applied.

13. Apparatus as claimed in Claim 12 characterised in that said means for providing an error signal includes said period determination means, means for computing the average of said periods and means for providing said error signal if the last detected period does not match said average within a predetermined tolerance.

14. Apparatus as claimed in Claim 13 characterised in that said means for capturing the occluding pressure during stat mode operation includes means for temporarily capturing the occluding pressure prevailing upon the first oscillation within said predetermined range of amplitude during said progressive decrease in said occluding pressure, means for detecting the second oscillation within said range of amplitudes during said progressive decrease in said occluding pressure, means for computing the period between said first and second oscillations and means for rejecting said temporarily captured occluding pressure if said computed period does not match said average of said periods within said predetermined tolerance.

15. Apparatus as claimed in any one of claims 2 to 14 characterised in that said selection means 22 includes means for maintaining said control means in said normal mode absent manual actuation, and means for automatically returning said control means to said normal mode from said stat mode after a predetermined interval of stat mode operation.

16. Apparatus as claimed in any one of Claim 1 to 15 characterised in that said pressure application means includes a distensible member 26, means for holding said distensible member against said pressure application portion of said subject and means for retaining a fluid under pressure in said distensible member.

17. Apparatus as claimed in any one of claims 1 to 16 characterised in that said transducer means includes means 34 for monitoring the pressure of said fluid and generating a fluid pressure signal representative of said fluid pressure, said control means being operative in said normal mode to decrease said fluid pressure progressively through a normal mode range encompassing the systolic and diastolic blood pressures of said subject, whereby oscillations in said fluid pressure will indicate pulsatile blood flow in said portion of said subject, said signal processing means including means for detecting oscillations in said fluid pressure signal during normal mode operation.

18. A method of monitoring blood pressure of a subject characterised by the steps of:

(a) automatically monitoring both the systolic and diastolic blood pressures of said subject in a normal mode of operation; and

(b) interrupting said normal mode of operation in response to a manually applied actuation, and

(c) automatically determining only the systolic blood pressure of said subject in a stat mode of operation when said normal mode of operation is interrupted in response to said manually applied actuation.

19. A method as claimed in Claim 18, characterised in that said systolic blood pressure is determined in said stat mode by automatically applying an occluding pressure to a pressure application region of the body of said subject and automatically decreasing said occluding pressure from above said systolic pressure while detecting the presence or absence of a pulse in a region of said subject's body distal to said pressure application region.

20. A method as claimed in Claim 19, characterised in that said step of detecting the presence of absence of a pulse in said distal region includes the steps of generating a plethysmographic signal representing the volume of said distal region and detecting the presence or absence of oscillations in said plethysmographic signal while said occluding pressure is decreased in said stat mode.

21. A method as claimed in Claim 20, characterised in that the steps of amplifying said plethysmographic signal and adjusting the gain applied in said amplification step while said occluding presence is not applied so as to provide oscillations in said amplified plethysmographic signal within a predetermined range of magnitude, said step of detecting the presence or absence of oscillations in said plethysmographic signal including the step of detecting oscillations in said amplified plethysmographic signal within said predetermined range of magnitudes and rejecting oscillations in said amplified plethysmographic signal outside of said predetermined range of magnitudes while said occluding pressure is decreased in said stat mode.

22. A method as claimed in Claim 21, characterised by the steps of determining the average period of oscillations in said amplified plethysmographic signal while said occluding pressure is not applied, said step of detecting oscillations in said amplified plethysmographic signal including the step of rejecting oscillations in said amplified plethysmographic signal which do not conform to said average period within a predetermined tolerance.

23. A method of repetitively determining the systolic blood pressure of a subject characterised by the steps of applying an occluding pressure to a pressure application region of the body of said subject, automatically decreasing said occluding pressure from above said systolic pressure while detecting the presence or absence of a pulse in a distal region of said subject's body distal to said pressure application region, whereby said pulse will be interrupted while said occluding pressure is above said systolic pressure, capturing the occluding pressure prevailing when said pulse in said distal region resumes, providing said captured occluding pressure as said sys- tolic pressure, automatically increasing said occluding pressure to above said systolic pressure in response to said resumption of said pulse, and then repeating the foregoing steps.

24. A method as claimed in Claim 23, characterised in that said step of decreasing said occluding pressure includes the step of decreasing said occluding pressure at an average rate of at least about 3mm Hg/sec.

FIG. 1

EP 0 298 620 A1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | MEDICAL & BIOLOGICAL ENGINEERING & COMPUTING vol. 23, no. 5, September 1985, pages 459-967, Stevenage, Herts, GB; K. YAMAKOSHI et al.: "Long-term ambulatory monitoring of indirect arterial blood pressure using a volume-oscillometric method" * page 459, right-hand column, line 16 - page 461, right-hand column, line 42; figures 1-3 * | 1,3,6-9 | A 61 B 5/02 |
| A | EP-A-0 024 772 (BATTELLE MEMORIAL INSTITUTE) * page 7, line 24 - page 8, line 7; figure 2 * | 1,2,6,8 | |
| A | EP-A-0 212 967 (TRIPOD INDUSTRIES CO. LTD.) * abstract; page 10, line 4 - page 14, line 13; figures 1, 2, 6 * | 1,3,6 | |
| A | US-A-3 920 004 (NAKAYAMA) * column 3, line 31 - column 4, line 63; figures 1, 2 * | 1,2,6 | TECHNICAL FIELDS SEARCHED (Int. Cl.3)  A 61 B 5/00 |
| A | US-A-4 597 393 (YAMUKOSHI et al.) * column 2, line 30 - column 3, line 13; column 5, line 32 - column 7, line 41; figures 1, 8, 11, 12 * | 1,3,6-9 | |

The present search report has been drawn up for all claims

| Place of search | Date of completion of the search | Examiner |
|---|---|---|
| BERLIN | 27-09-1988 | WEIHS J.A. |